Europäisches Patentamt

European Patent Office

Office européen des brevets

(19)

(11) Veröffentlichungsnummer: **0 176 769 B1**

(12) **EUROPÄISCHE PATENTSCHRIFT**

(45) Veröffentlichungstag der Patentschrift: **27.05.92**

(21) Anmeldenummer: **85110881.1**

(22) Anmeldetag: **29.08.85**

Die Akte enthält technische Angaben, die nach dem Eingang der Anmeldung eingereicht wurden und die nicht in dieser Patentschrift enthalten sind.

(51) Int. Cl.⁵: **A61K 45/06**, A61K 31/725, A61K 37/54, //(A61K37/54, 31:725)

(54) **Carcinom-Therapeutikum.**

(30) Priorität: **05.09.84 DE 3432661**

(43) Veröffentlichungstag der Anmeldung: **09.04.86 Patentblatt 86/15**

(45) Bekanntmachung des Hinweises auf die Patenterteilung: **27.05.92 Patentblatt 92/22**

(84) Benannte Vertragsstaaten: **AT BE CH DE FR GB IT LI LU NL SE**

(56) Entgegenhaltungen:
**DE-A- 1 951 822**
**DE-A- 3 220 326**

(73) Patentinhaber: **Landsberger, Albert Prof.-Dr. Lerchenweg 6 W-6901 Nussloch(DE)**

(72) Erfinder: **Landsberger, Albert Prof.-Dr. Lerchenweg 6 W-6901 Nussloch(DE)**

(74) Vertreter: **Patentanwälte Grünecker, Kinkeldey, Stockmair & Partner Maximilianstrasse 58 W-8000 München 22(DE)**

EP 0 176 769 B1

**Beschreibung**

Die Erfindung betrifft eine therapeutische Zubereitung, enthaltend Glycosaminoglycanpolysulfate und mindestens ein Zytostatikum neben üblichen Trägern und Begleitsubstanzen.

Die klassischen Behandlungsmethoden bei bösartigen Tumorerkrankungen beschränken sich im wesentlichen auf chirurgische und/oder radiologische Behandlungen sowie auf die Anwendung von Zytostatika. Die Nachteile der radiologischen und zytostatischen Behandlungsverfahren sind bekannt. Die heute übliche klinische Behandlungsweise mit zumeist mehreren Zytostatika vernichtet Tumorzellen fast ausschließlich in der Teilungsphase. In der sogenannten Ruhephase werden Krebszellen durch Zytostatika dagegen kaum angegriffen. Bei den meisten Krebsarten aber befinden sich die Zellen überwiegend in der Ruhephase. Dementsprechend sind gute Erfolge durch eine zytostatische Chemotherapie in ihrer üblichen klinischen Behandlungsweise nur bei denjenigen Tumoren beschrieben, die eine hohe Teilungsrate haben, z.B. bei Hodgkin- und Non-Hodgkin-Lymphomen und bestimmten Leukämieformen. Die Anwendung der zytostatischen Chemotherapie wird weiterhin durch ihre erheblichen Nebenwirkungen eingeengt, die vor allem dadurch bedingt werden, daß Zytostatika auch gesunde Gewebszellen angreifen.

Es ist aus "Journal of Medicinal Chemistry, 1974, Vol. 17, No. 12, S.1335" bekannt, die Behandlung von Tumorerkrankungen mit Zytostatika durch gleichzeitige Zugabe von Heparin in der Wirksamkeit zu steigern. Es wurde beobachtet, daß Patienten, die bei der Behandlung mit Zytostatika ohne Heparin keine Reaktion zeigten, besser reagierten, wenn begleitend Heparin verwendet wurde. Auch mit dem Heparin hatte man jedoch immer noch keine Substanz gefunden, die eine signifikante Wirkungssteigerung der Zytostatika ergab. Außerdem vermochte Heparin nicht,gesunde Gewebszellen vor den Zytostatika zu schützen, also die Nebenwirkungen zu verringern. Im Gegenteil traten bei dieser Therapieform zusätzlich innere Blutungen auf, die die Anwendung des Heparins zusammen mit Zytostatika erschweren.

Aus der Deutschen Offenlegungsschrift 32 20 326 ist bekannt, die Behandlung von Tomorerkrankungen mit Zytostatika durch gleichzeitige Zugabe von anderen Glycosaminoglycanpolysulfaten als Heparin signifikant zu verbessern. Diese therapeutische Zubereitung vermag die Tumorzellen selektiv zu "öffnen" und zytotoxisch bzw. zytolytisch wirksame Stoffe gezielt einzuschleusen. Gesunde Gewebszellen bleiben dadurch von den in wesentlich geringeren Dosen angewandten Zytostatika weitgehend oder völlig unversehrt, so daß die schweren Nebenwirkungen, wie sie in der sonst üblichen klinischen Anwendungsweise der zytostatischen Chemotherapie auftreten, erheblich vermindert oder aufgehoben werden. Auch bei dieser Carcinom-Therapie hat sich jedoch gezeigt, daß immer noch ein gewisser Teil der Tumorzellen in der Ruhephase nicht angegriffen und zerstört wird.

Der Erfindung liegt daher die Aufgabe zugrunde, eine therapeutische Zubereitung zur Verfügung zu stellen, die eine vor allem in der Ruhephase der Tumorzelle verstärkte Wirkung und damit insgesamt auch eine erhöhte Selektivität besitzt. Sie soll Tumorzellen in erhöhtem Maße gezielt und permanent, also sowohl in Teilungs- als auch in Ruhephase, angreifen und vernichten.

Diese Aufgabe wird, ausgehend von einer therapeutischen Zubereitung laut Oberbegriff des Hauptanspruchs, durch die im kennzeichnenden Teil desselben angegebenen Merkmale gelöst.

Glycosaminoglycanpolysulfate, auch als sulfatierte Polyanionen oder früher als Mucopolysaccharidpolyschwefelsäureester bezeichnet, sind aus der Therapie verschiedener Erkrankungen bekannt. Zu dieser Verbindungsklasse gehören z.B. die Heparine, deren gerinnungsphysiologische Wirkung seit langem genutzt wird. Andere Verbindungen dieser Substanzklasse werden bei der Behandung von Hyperlipidämien und Hypercholesterinämien (z.B. Heparine und Heparinoide) oder als Antiarthrotikum (z.B. Knorpelextrakte und Chondroitinpolysulfat) verwendet. Weiterhin war von den betrachteten Substanzen eine ausgeprägte und spezifische Inhibition von Enzymsystemen bekannt (Schaffrath et al. in :Hoppe-Seylers Z.Physiol.Chem. 357, 499 (1976)). Bei Untersuchungen an Zellkulturen wurde nun gefunden, daß maligne Zellen die erfindungsgemäßen Gemische von Glycosaminoglycanpolysulfaten stärker aufnehmen und speichern als benigne Zellen.

Die verstärkte Aufnahme von Glycosaminoglycanpolysulfaten führt zu einer Hemmung von biosynthetischen Leistungen im Zellstoffwechsel maligner Zellen. Somit tritt eine synergistische Wirkung zur Wirkung der gleichzeitig verwendeten Zytostatika auf, und letztere können daher in Konzentrationen angewandt werden, die die benignen Zellen nicht oder nur geringfügig beeinträchtigen.

Als Zytostatika können erfindungsgemäß z.B. alkylierende Zytostatika, wie Stickstofflost-Derivate und Äthyleniminderivate, Antimetaboliten, wie Methotrexat, Antagonisten von Purin- und Pyrimidin-Basen, zytostatisch wirksame Antibiotika und Alkaloide, wie Cholchicin oder Vincristin, eingesetzt werden.

Als sehr vorteilhaft hat sich weiterhin erwiesen, zu der therapeutischen Zubereitung noch mindestens ein proteolytisches und/oder lipolytisches Enzym, z.B. Trypsin, zuzusetzen. Der damit verbundene Effekt war außerordentlich überraschend. Aus dem allgemeinen biochemischen Fachwissen ist nämlich bekannt, daß ein proteolytisches Enzym, wie z.B. Trypsin, durch Interaktion mit Glycosaminoglycanpolysulfaten die Wirkung beider Substanzen gewissermaßen aufhebt. Aufgrund dieser Erkenntnis war dem Fachmann der Weg verlegt, im Sinne der vorliegenden Erfindung weiterzuarbeiten. Durch noch nicht vollständig verstandene Wechselwirkungen zwischen allen erfindungsgemäß verwendeten Substanzen tritt jedoch, wie die Versuche in der vorliegenden Patentanmeldung zeigen, in ganz unerwarteter Weise kein hemmender, sondern ganz im Gegenteil ein synergistischer Effekt ein.

Trypsin ist in der Literatur als zytolytische Substanz beschrieben. Es greift selektiv die maligne Zelle an, die durch zusätzliche Applikation des erfindungsgemäßen Glycosaminoglycanpolysulfatgemischs bereits geschädigt ist. Ein Zusatz von mindestens einem proteolytischen und/oder lipolytischen Enzym, wie z.B. Trypsin, ist deshalb bedeutsam, weil es auch innerhalb eines gleichen Tumorzellenstammes biologisch resistente Arten gibt. Solche resistenten Tumorzellen werden durch das Enzym, bzw. die Enzyme angedaut, um das Zytostatikum wirksam werden zu lassen. Das Enzym bzw. die Enzyme verstärken daher die selektive Wirkung der erfindungsgemäßen Zubereitung auf maligne Zellen und ermöglicht so die gezielte Anwendung eines Zytostatikums oder mehrerer Zytostatika in geringen Dosen, die bei alleiniger Verwendung in den viel höheren therapeutisch wirksamen Dosen alle, also auch benigne Zellen, schädigen. Durch die Glycosaminoglycanpolysulfatgemische, gegebenenfalls zusammen mit mindestens einem proteolytischen und/oder lipolytischen Enzym, wie z.B. Trypsin, werden die Tumorzellen selektiv "geöffnet", wodurch zytotoxisch bzw. zytolytisch wirksame Stoffe gezielt eingeschleust werden, wobei auch das bzw. die Enzyme zytolytisch wirken.

Die Versuche haben gezeigt, daß eine therapeutische Zubereitung, die mindestens zwei Glycosaminoglycanpolysulfate verschiedenen Molekulargewichts und mindestens ein Zytostatikum und gegebenenfalls noch mindestens ein proteolytisches und/oder lipolytisches Enzym, wie z.B. Trypsin, enthält, noch deutlich wirksamer Tumorzellen angreift und vernichtet als die in der Deutschen Offenlegungsschrift 32 20 326 definierten therapeutischen Zubereitung. Histologische Untersuchungen haben ergeben, daß die erfindungsgmäße Kombination Tumorzellen gerade in der Ruhepause erheblich stärker anzugreifen und zu vernichten in der Lage ist als die genannte ,früher beschriebene therapeutische Zubereitung, die Tumorzellen nur teilweise in der Ruhephase erfolgreich bekämpft. Da, wie eingengs bereits erläutert, die Zellen der meisten Krebsarten sich überwiegend in der Ruhephase befinden, ist diese neuartige Wirkung der erfindungsgemäßen Kombination von größter Bedeutung. Somit sind die Zellen voraussichtlich sämtlicher Krebsarten permanent, d.h. in Teilungsphase und Ruhephase, verstärkt bekämpfbar.

Diese verstärkte Wirkung der erfindungsgemäßen Kombination bewirkt zusätzlich eine weiter erhöhte Selektivität, da die zytotoxisch bzw. zytolytisch wirksamen Substanzen kaum noch oder überhaupt nicht mehr an Tumorzellen in der Ruhephase scheitern und daraufhin gesunde Zellen anfallen können. Die Nebenwirkungen der zytostatischen Chemotherapie können also noch weiter reduziert werden, zumal da auch die Dosen der Zytostatika weiter verringert werden können.

Ein weiterer neuartiger Vorteil der erfindungsgemäßen Kombination ist die erhebliche Verminderung der antigenen Reaktionen auf die hochmolekularen Glycosaminoglycanpolysulfate durch den Zusatz von niedermolekularen Glycosaminoglycanpolysulfaten. Eine alleinige Verwendung niedermolekularer Glycosaminoglycanpolysulfate ist jedoch deshalb nicht angebracht, weil deren carcinomtherapeutische Wirkung nicht mit derjenigen der höhermolekularen Glycosaminoglycanpolysulfaten zu vergleichen ist.

Es hat sich herausgestellt, daß die erfindungsgemäßen Gemische von Glycosaminoglycanpolysulfaten sich insbesondere dadurch auszeichnen, daß eine Wirkung auch dann auftritt, wenn die Tumoren bereits eine hohe Zellzahl aufweisen. Obwohl eine abschließende wissenschaftliche Erklärung für diese gesteigerte selektive Verhaltensweise fehlt, wird vermutet, daß die spezielle Art und der Grad der Sulfatierung für das beobachtete Phänomen verantwortlich sind.

Erfindungsgemäß weisen die hochmolekularen Glycosaminoglyconpolysulfate des Gemisches ein Molekulargewicht von mehr als 5000 bis 15000, bevorzugt im Bereich von 8000 bis 12000 auf. Die niedermolekularen Glycosaminoglycanpolysulfate weisen ein Molekinlargewicht im Bereich von 1500 bis 5000, bevorzugt im Bereich von 1500 bis 3000 auf.

Als besonders vorteilhaft hat sich als niedermolekulares Glycosaminoglycanpolysulfat Pentosanpolysulfat, insbesondere als Alkali-Salz, vor allem als Natrium-Salz,erwiesen.

Das Mengenverhältnis von Glycosaminoglycanpolysulfaten zu Zytostatika liegt zwischen 10 : 1 und 1 : 10, bezogen auf Gewichtsteile der Wirksubstanzen.

Die Menge an Zytostatikum hängt von der Art des verwendeten Zytostatikums ab.

Zur Herstellung von Arzneimitteln werden Zubereitungen in an sich bekannter Weise mit geeigneten Pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Oel, wie z.B. Olivenöl, suspendiert oder gelöst.

Die Substanzen der Zubereitungen können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Fettsäuren (wie Stearinsäure), Gelatine,Agar-Agar, Calciumphosphat, Magnesiumstearat, tierische und pflanzliche Fette oder feste hochmolekulare Polymere (wie Polyethylenglykole). Für die orale Applikation geeignete Zubereitungen können gewünschtenfalls Geschmacks- und Süßstoffe enthalten.

Die therapeutische Einzeldosis der therapeutischen Zubereitung beträgt zwischen 5 und 500 mg.

Die Erfindung wird durch den nachfolgenden Versuch näher erläutert. Es wurden die folgenden pharmakologisch aktiven Substanzen verwendet:

a) Glycosaminoglycanpolysulfate:

1. Mucopolysaccharidpolyschwefelsäureester (MPS)
Molekulargewicht 10.000

2. Natrium-Pentosanpolysulfat (PSS)
Molekulargewicht 2.000

b) Zytostatikum: Mitomycin

c) Enzym: Trypsin ( reine Darstellung)

Versuch

Es wurden 60 gleichgeschlechtliche Ratten (Wistar) in drei Gruppen randomisiert.

Auf jedes einzelne Tier wurden 10 000 000 Zellen des Yoshida-Ascites-Carcinom (Hämorrhagisch) übertragen. Die einzelnen Gruppen wurden mit folgenden Wirkstoffen behandelt:

Gruppe A:     MPS + PSS + Mitomycin

Gruppe B:     MPS + PSS + Mitomycin + Trypsin

Gruppe C:     Kontrolle

Die Wirksubstanzen wurden den Ratten in folgenden Dosen pro kg Ratte gegeben:

| | |
|---|---|
| MPS | 5 mg/kg Ratte |
| PSS | 5 mg/kg Ratte |
| Mitomycin | 0,4 mg/kg Ratte |
| Trypsin | 0,2 mg/kg Ratte |

Therapiebeginn 48 Stunden nach Tumorübertragung. Tiere der Gruppe A erhielten insgesamt 4 Injektionen, jeweils im Abstand von 48 Stunden.

Tiere der Gruppe B erhielten insgesamt 3 Injektionen, ebenfalls im Abstand von jeweils 48 Stunden. 3 Wochen nach der letzten Injektion wurde der Versuch ausgewertet. Die Ergebnisse sind in der Tabelle zusammengestellt:

Tabelle

| Gruppe mit 20 Tieren | lebend | davon tumorfrei | tot | davon mit Tumor |
|---|---|---|---|---|
| A | 19 | 19 | 1 | 0 |
| B | 20 | 20 | 0 | 0 |
| C | 0 | 0 | 20 | 20 |

Der Versuch zeigt, daß in Gruppe B mit 3 Injektionen die gleiche Wirkung erreicht wurde wie in Gruppe A mit 4 Injektionen. Da es aber Resistenzunterschiede zwischen den verschiedenen Tumorarten und -Zellen gibt, kann auf keine der angeführten wirksamen Kombinationen verzichtet werden.

Es hat sich ergeben, daß MPS und PSS, weder allein noch kombiniert, einen genügenden zytostatischen Effekt haben. Gleiches gilt für Mitomycin und Trypsin.

**Patentansprüche**
**Patentansprüche für folgende Vertragsstaaten : BE, CH, DE, FR, GB, IT, LI, LU, NL, SE.**

1. Therapeutische Zubereitung, enthaltend Glycosaminoglycanpolysulfat und mindestens ein Zytostatikum neben üblichen Trägern und Begleitsubstanzen, **dadurch gekennzeichnet**, daß das Glycosaminoglycanpolysulfat ein Gemisch von Glycosaminoglycanpolysulfat mit einem Molekulargewicht von mehr als 5000 bis 15000 und von Pentosanpolysulfat mit einem Molekulargewicht von 1500 bis 5000 ist.

2. Therapeutische Zubereitung nach Anspruch 1, **dadurch gekennzeichnet**, daß sie zusätzlich mindestens ein proteolytisches und/oder lipolytisches Enzym enthält.

3. Therapeutische Zubereitung nach Anspruch 1 oder 2, **dadurch gekennzeichnet**, daß das Glycosaminpolysulfat ein Gemisch von Glycosaminoglycanpolysulfat mit einem Molekulargewicht von 8000 bis 12000 und von Pentosanpolysulfat mit einem Molekulargewicht von 1500 bis 3000 ist.

4. Therapeutische Zubereitung nach mindestens einem der Ansprüche 1 bis 3, **dadurch gekennzeichnet**, daß das Pentosanpolysulfat Natriumpentosanpolysulfat ist.

5. Therapeutische Zubereitung nach mindestens einem der Ansprüche 2 bis 4, **dadurch gekennzeichnet**, daß das zusätzliche Enzym Trypsin ist.

6. Therapeutische Zubereitung nach mindestens einem der Ansprüche 1 bis 5, **dadurch gekennzeichnet**, daß das Mengenverhältnis zwischen dem Gemisch von Glycosaminoglycanpolysulfaten und Zytostatika im Bereich von 1 : 10 bis 10 : 1 liegt.

**Patentansprüche für folgenden Vertragsstaat : AT**

1. Verfahren zur Herstellung einer therapeutischen Zubereitung, enthaltend Glycosaminoglycanpolysulfat, **dadurch gekennzeichnet**, daß man als Glycosaminoglycanpolysulfat ein Gemisch von Glycosaminoglycanpolysulfat mit einem Molekulargewicht von mehr als 5000 bis 15000 und von Pentosanpolysulfat mit einem Molekulargewicht von 1500 bis 5000 mit mindestens einem Zytostatikum, üblichen Trägern und Begleitsubstanzen kombiniert.

2. Verfahren nach Anspruch 1, **dadurch gekennzeichnet**, daß man zusätzlich mindestens ein proteolytisches und/oder lipolytisches Enzym kombiniert.

3. Verfahren nach Anspruch 1 oder 2, **dadurch gekennzeichnet**, daß man als Glycosaminopolysulfat ein Gemisch von Glycosaminoglycanpolysulfat mit einem Molekulargewicht von 8000 bis 12000 und von Pentosanpolysulfat mit einem Molekulargewicht von 1500 bis 3000 verwendet.

4. Verfahren nach mindestens einem der Ansprüche 1 bis 3, **dadurch gekennzeichnet**, daß man als Pentosanpolysulfat Natriumpentosanpolysulfat verwendet.

5. Verfahren nach mindestens einem der Ansprüche 2 bis 4, **dadurch gekennzeichnet**, daß man als zusätzliches Enzym Trypsin verwendet.

6. Verfahren nach mindestens einem der Ansprüche 1 bis 5, **dadurch gekennzeichnet**, daß das Mengenverhältnis zwischen dem Gemisch von Glycosaminoglycanpolysulfaten und Zytostatika im Bereich von 1:10 bis 10:1 liegt.

**Claims**
**Claims for the following Contracting States : BE, CH, DE, FR, GB, IT, LI, LU, NL, SE.**

1. A therapeutic preparation, containing glycosaminoglycan polysulphate and at least one antitumour agent in addition to conventional carriers and accompanying substances, characterised in that the glycosaminoglycan polysulphate is a mixture of glycosaminoglycan polysulphate having a molecular weight of more than 5000 to 15000 and of pentosan polysulphate having a molecular weight of 1500 to 5000.

**2.** A therapeutic preparation according to Claim 1, characterised in that it additionally contains at least one proteolytic and/or lipolytic enzyme.

**3.** A therapeutic preparation according to Claim 1 or 2, characterised in that the glycosaminopolysulphate is a mixture of glycosaminoglycan polysulphate having a molecular weight of 8000 to 12000 and of pentosan polysulphate having a molecular weight of 1500 to 3000.

**4.** A therapeutic preparation according to at least one of Claims 1 to 3, characterised in that the pentosan polysulphate is sodium pentosan polysulphate.

**5.** A therapeutic preparation according to at least one of Claims 2 to 4, characterised in that the additional enzyme is trypsin.

**6.** A therapeutic preparation according to at least one of Claims 1 to 5, characterised in that the quantitative ratio between the mixture of glycosaminoglycan polysulphates and anti-tumour agents is in the range of 1 : 10 to 10 : 1.

**Claims for the following Contracting State : AT**

**1.** A method for producing a therapeutic preparation, containing glycosaminoglycan polysulphate, characterised in that a mixture of glycosaminoglycan polysulphate having a molecular weight of more than 5000 to 15000 and of pentosan polysulphate having a molecular weight of 1500 to 5000 with at least one anti-tumour agent, conventional carriers and accompanying substances is combined as the glycosaminoglycan polysulphate.

**2.** A method according to Claim 1, characterised in that additionally at least one proteolytic and/or lipolytic enzyme is combined.

**3.** A method according to Claim 1 or 2, characterised in that a mixture of glycosaminoglycan polysulphate having a molecular weight of 8000 to 12000 and of pentosan polysulphate having a molecular weight of 1500 to 3000 is used as the glycosaminopolysulphate.

**4.** A method according to at least one of Claims 1 to 3, characterised in that sodium pentosan polysulphate is used as the pentosan polysulphate.

**5.** A method according to at least one of Claims 2 to 4, characterised in that trypsin is used as the additional enzyme.

**6.** A method according to at least one of Claims 1 to 5, characterised in that the quantitative ratio between the mixture of glycosaminoglycan polysulphates and antitumour agents is in the range of 1 : 10 to 10 : 1.

**Revendications**
**Revendications pour les Etats contractants suivants : BE, CH, DE, FR, GB, IT, LI, LU, NL, SE.**

**1.** Préparation thérapeutique contenant du polysulfate de glycosaminoglycane et au moins un cytostatique, ainsi que des supports et adjuvants usuels, caractérisée en ce que le polysulfate de glycosaminoglycane est un mélange de polysulfate de glycosaminoglycane ayant une masse moléculaire dans une gamme allant de 5000 jusqu'à 15000 et de polysulfate de pentosane ayant une masse moléculaire de 1500 à 5000.

**2.** Préparation thérapeutique selon la revendication 1, caractérisée en ce qu'elle contient en outre au moins une enzyme protéolytique et/ou lipolytique.

**3.** Préparation thérapeutique selon la revendication 1 ou 2, caractérisée en ce que le polysulfate de glycosaminoglycane est un mélange de polysulfate de glycosaminoglycane ayant une masse moléculaire de 8000 à 12000 et de polysulfate de pentosane ayant une masse moléculaire de 1500 à 3000.

6

**4.** Préparation thérapeutique selon au moins l'une des revendications 1 à 3, caractérisée en ce que le polysulfate de pentosane est le polysulfate de pentosane sodique.

**5.** Préparation thérapeutique selon au moins l'une des revendications 2 à 4, caractérisée en ce que l'enzyme additionnelle est la trypsine.

**6.** Préparation thérapeutique selon au moins l'une des revendications 1 à 5, caractérisée en ce que les proportions du mélange de polysulfate de glycosaminoglycane et de cytostatique sont dans la gamme de 1:10 à 10:1.

**Revendications pour l'Etat contractant suivant : AT**

**1.** Procédé pour l'obtention d'une préparation thérapeutique contenant du polysulfate de glycosaminoglycane, caractérisé en ce que comme polysulfate de glycosaminoglycane on utilise un mélange de polysulfate de glycosaminoglycane ayant une masse moléculaire dans une gamme allant de plus de 5000 jusqu'à 15000, et de polysulfate de pentosane ayant une masse moléculaire de 1500 à 5000, en combinaison avec au moins un cytostatique, et des supports et adjuvants usuels.

**2.** Procédé selon la revendication 1, caractérisé en ce que la combinaison contient en outre au moins une enzyme protéolytique et/ou lipolytique.

**3.** Procédé selon la revendication 1 ou 2, caractérisé en ce que comme polysulfate de glycosaminoglycane on utilise un mélange de polysulfate de glycosaminoglycane ayant une masse moléculaire de 8000 à 12000 et de polysulfate de pentosane ayant une masse moléculaire de 1500 à 3000.

**4.** Procédé selon au moins l'une des revendications 1 à 3, caractérisé en ce que comme polysulfate de pentosane, on utilise le polysulfate de pentosane sodique.

**5.** Procédé selon au moins l'une des revendications 2 à 4, caractérisé en ce que comme enzyme additionnelle, on utilise la trypsine.

**6.** Procédé selon au moins l'une des revendications 1 à 5, caractérisé en ce que les proportions du mélange de polysulfate de glycosaminoglycane et de cytostatique sont dans la gamme de 1:10 à 10:1.